# EUROPEAN PATENT SPECIFICATION

(11) **EP 1 221 479 B1**
(45) Date of publication and mention of the grant of the patent: **22.11.2006**
(21) Application number: 00961196.3
(22) Date of filing: 22.09.2000
(51) Int. Cl.: C12N 9/96, C12N 9/74

(54) **METHOD FOR STABILIZING THROMBIN**
VERFAHREN ZUR STABILISIERUNG VON THROMBIN
PROCEDE DE LA STABILISATION DE LA THROMBINE

(30) Priority: 27.09.1999 JP 27209299; 13.07.2000 JP 2000212924
(43) Date of publication of application: 10.07.2002
(73) Proprietor: SYSMEX CORPORATION, Kobe-shi, Hyogo 651-0073 (JP)
(72) Inventor: OKUDA, Masahiro, c/o International Reagents Corp., Kobe-shi, Hyogo 651-22 (JP); KIKUKAWA, Norihiro, c/o Internat. Reagents Corp., Kobe-shi, Hyogo 651-2241 (JP)
(74) Representative: Forstmeyer, Dietmar
(86) International application number: PCT/JP2000/006513
(87) International publication number: WO 2001/023536

(56) References cited:
- EP-A- 0 444 692
- EP-A- 0 478 827
- EP-A1- 0 443 724
- EP-A1- 0 570 354
- EP-A1- 0 823 475
- EP-A2- 0 040 799
- EP-A2- 0 221 700
- EP-A2- 0 302 754
- EP-A2- 0 699 909
- WO-A-97/37694
- WO-A1-96/26269
- JP-A- 513 899
- JP-A- 56 039 782
- US-A- 4 409 334
- US-A- 5 304 372
- US-A- 5 506 127
- CHABBAT J ET AL: "PROPERTIES OF A NEW FIBRIN GLUE STABLE IN LIQUID STATE" THROMBOSIS RESEARCH, TARRYTOWN, NY, US, vol. 76, no. 6, 15 December 1994 (1994-12-15), pages 525-533, XP000884761 ISSN: 0049-3848
- DATABASE BIOSIS [Online] BIOSCIENCES INFORMATION SERVICE, PHILADELPHIA, PA, US; 1976 MACHOVICH R: "EFFECT OF CALCIUM PHOSPHATE ON THROMBIN AND ON ITS SENSITIVITY TO HEPARIN AND ANTI THROMBIN III" Database accession no. PREV197763010265 XP002276457 & THROMBOSIS RESEARCH, vol. 9, no. 2, 1976, pages 123-131, ISSN: 0049-3848

## Description

### Technical Field

The present invention relates to a means for the stabilization of thrombin and to a composition containing the stabilized thrombin. More particularly, it relates to a means for the stabilization of a thrombin reagent used in the field of clinical tests and to a reagent containing the stabilized thrombin.

### Background of the Invention

Thrombin which is an enzyme for conversion of fibrinogen to fibrin plays a very important role in a process of blood coagulation reaction. In the field of clinical laboratory tests, it is frequently used for measuring the concentration of fibrinogen using thrombin and for measuring the activity of thrombin-inhibitors such as anti-thrombin activity, hirudine activity and chemical synthesis inhibitor.

However, thrombin is unstable in general and, for its preservation for a long period, it is usually preserved after subjecting to a freeze-drying treatment.

Attempts for the stabilization of thrombin have been abundantly carried out up to now. They are mentioned, for example, in Japanese Patent Laid-Open No. 39782/1981, Japanese Patent Publication No. 76292/1991, Japanese Patent Laid-Open Nos. 93536/1989, 53732/1990, 40433/1989, 33799/1995 and 328436/1997, Japanese Patent Publication No. 13750/1996, Japanese Patent Laid-Open No. 165604/1995, Japanese Patent Publication No. 64747/1995, Japanese Patent Laid-Open Nos. 192723/1988 and 320683/1992, Japanese Patent Publication No. 26956/1983 and Japanese Patent Laid-Open Nos. 52267/1998, 112721/1985 and 68607/1981.

US-A-5 304 372 describes a process for preparing a human thrombin concentrate intended for therapeutic use.

EP-A-0 478 827 discloses a stabilized thrombin, its preparation and use as a thrombin reagent.

EP-A-0 444 692 discloses a stable thrombin composition for oral administration.

US-A-4 409 334 describes a stabilized thrombin preparation in solid or dissolved form.

Chabbat J et al., Thrombosis Res., U.S., Vol. 76, No. 6, pp. 525-533, 1994 describes a pasteurized preparation of fibrin glue comprising highly purified human thrombin and fibrinogen concentrate.

According to the abstract available in the BIOSIS database (online), BIOSCIENCES INFORMATION SERVICE, Philadelphia, PATENTANMELDUNG, US, Machovich R, Thrombosis Res., Vol. 9, No. 2, pp. 123-131, 1976 describes the effect of calcium phosphate on thrombin and on its sensitivity to heparin and antithrombin-III.

EP-A-0 221 700 discloses a further thrombin preparation.

EP-A-0 699 909 describes a method for determining fibrinogen and a reagent for determination thereof.

However, any of them is not perfect as a method for the stabilization of thrombin and, particularly for use as a diagnostic agent for clinical laboratory tests, any of them does not show a sufficient stabilization.

In the meanwhile, thrombin used as a measuring reagent is prepared in such a manner that thrombin usually derived from human being or animals is added to a solution containing buffer or stabilizer to give an aimed titer and is preserved and used as a solution or a freeze-dried product. The thrombin reagent prepared as such is used in various objects as mentioned already and, in any of the cases, activity of thrombin itself is measured finally whereby the aimed object is achieved.

With regard to measurement of the thrombin activity, a measuring method using an artificially synthesized substrate and a measuring method using fibrinogen which is a natural substrate are commonly used. For the former, colorimetric analysis is usual while, for the latter, analysis of coagulation time is common. In the latter method for analysis of coagulation time, there has been known a method where coagulation time is determined by detecting the changes in scattering light or transmission light and a physical method where coagulation time is determined by detecting the fact that spheres become unable to move due to formation of coagulation.

The biggest problem in measuring the coagulation time in the above-mentioned detecting methods is that the reproducibility of the measurement is poor. Especially, the conversion reaction of fibrinogen to fibrin using thrombin is a reaction of transition from a liquid phase reaction to a solid phase one and it has been known to be greatly affected by the reaction condition such as pH and ionic strength. As a result of the influence of such reaction conditions, reproducibility is poor. Under such circumstances, there has been a brisk demand for construction of a measuring system using liquid and stable thrombin, being unaffected by the reaction condition and having a good reproducibility.

### Disclosure of the Invention

An object of the present invention is to provide a means for the stabilization of thrombin; a composition containing thrombin which is stabilized and has a good reproducibility for the measurement and a reagent using the said composition.

The present inventors have carried out an intensive investigation and, as a result, they have found that, when calcium ion or a mixture of calcium ion and water-soluble organic acid is contained in thrombin, inactivation of thrombin can be prevented in all of the cases of a solution state, a freeze-dried product and a solution prepared by reconstituting after a freeze-drying treatment. They have further found that surfactant, protein, etc. have a stabilizing effect to thrombin in addition to calcium ion and water-soluble organic acid. It has been also found that a thrombin reagent having a good reproducibility in the measurement can be prepared by such a way that one or more of high-molecular polysaccharides or synthetic polymers is/are mixed therewith and contained therein in addition to the above-mentioned substances showing a stabilizing effect. As a result, the present invention has been achieved.

Thus, the present invention comprises a use and a method in accordance with the attached claims. Preferred embodiments of the invention are the subject matter of the dependent claims.

Further, there is disclosed:
(1) a means for the stabilization of thrombin, characterized in that, effective amount(s) in terms of thrombin-stabilization of one or more compound(s) having a thrombin-stabilizing function selected from calcium ion, surfactant and protein is/are contained;
(2) the means for the stabilization of thrombin according to the above (1), wherein effective amount(s) in terms of thrombin-stabilization of one or more compound(s) having a thrombin-stabilizing function selected from calcium ion, surfactant and protein and, in addition, a water-soluble organic acid having a thrombin-stabilizing action are contained;
(3) the means for the stabilization of thrombin according to the above (2), wherein at least calcium ion and water-soluble organic acid are jointly used and effective amounts thereof in terms of thrombin-stabilization are contained therein;
(4) a thrombin-containing composition where at least one of the stabilization means for thrombin of from the above (1) to (3) is introduced;
(5) the thrombin-containing composition according to the above (4), wherein an effective amount of a substance having a measurement-reproducible improvement-function which is compounded of one or more being selected from high-molecular polysaccharides and/or synthetic polymers is contained with an object of securing the reproducibility of measurement of activity of the substance to be tested using a reagent containing thrombin;
(6) the thrombin-containing composition according to the above (4) or (5), wherein the composition is liquid;
(7) a method for the manufacture of a thrombin-containing composition into which at least one of the stabilizing means for thrombin described in the above (1)-(6) is introduced; and
(8) a method for the measurement using a thrombin-containing composition into which at least one of the stabilizing means for thrombin described in the above (1)-(6) is introduced is used as a reagent.

### Best Mode for Carrying Out the Invention

The use for improving preservation stability of thrombin in accordance with the present invention and the method for stabilizing thrombin in a thrombin reagent will be illustrated in more detail as hereunder. The use for improving preservation stability of thrombin according to the present invention is characterized in that nonionic surfactant and calcium ion are used in a thrombin reagent for clinical laboratory tests. In addition, a compound which is a water-soluble organic acid and has a thrombin-stabilizing function is further and jointly used. The amount effective in terms of stabilization of the compound having a stabilizing function means an amount by which stability is improved and, although there is no particular limitation therefor, it for example means an amount of the compound having a stabilizing function to such an extent that at least 50%, preferably at least 60% or, more preferably, at least 70% of activity as compared with the thrombin activity immediately after preparation of the thrombin solution is maintained when a solution containing thrombin is preserved at 25°C for six months. Here, the term "a means" stands for a method and/or a medium used for achieving the object.

### (Thrombin)

The thrombin which is to be stabilized by the stabilizing means for thrombin in accordance with the present invention may be any of that derived from animals, derived from human plasma and prepared by a genetic engineering means. Concentration of thrombin in a solution in the thrombin-containing composition into which the said stabilizing means is introduced is not particularly limited but its activity may be just adjusted to the aimed activity value.

### (Calcium Ion)

With regard to calcium ion used for a stabilizing means for thrombin, there may be exemplified water-soluble calcium compounds such as calcium chloride, calcium lactate, calcium gluconate, calcium glucuronate and calcium tartrate. With regard to such calcium compounds, one of them or two or more thereof may be selected and used.

The effective amount for the stabilization of calcium compound to thrombin may be an amount for just improving the stability and, although there is no particular limitation, the final concentration of the compound when thrombin is in the dissolved liquid state is preferably from 5 mM to 100 mM and, more preferably, from 10 mM to 50 mM.

### (Organic Acids)

With regard to the organic acid used as a stabilizing means for thrombin, a water-soluble one is used and there may be exemplified formic acid, acetic acid, propionic acid, butyric acid, valeric acid, oxalic acid, malonic acid, succinic acid, gluconic acid, lactic acid, glucuronic acid, glycolic acid, tartaric acid, malic acid, citric acid, tartaric acid, glutaric acid, aminoacetic acid and aminocaproic acid. Such an organic acid may be either a free acid or a salt thereof. One of them or two or more of them may be selected and used.

The effective amount for the stabilization of the organic acid to thrombin may be an amount for just improving the stability and, although there is no particular limitation, the final concentration of the organic acid when thrombin is in the dissolved liquid state is preferably from 10 mM to 500 mM and, more preferably, from 50 mM to 200 mM.

### (Surfactant)

With regard to the surfactant, there may be exemplified anionic surfactants such as sodium dodecylsulfate, sodium dodecylsulfonate, sodiumdodecyl-N-sarcosinate, sodiumcholate, sodium deoxycholate and sodium taurodeoxycholate; cationic surfactants such as cetyltrimethylammonium bromide, tetradecylammonium bromide and dodecylpyridinium chloride; amphoteric surfactants such as 3-[(3-cholamidopropyl)dimethylammonio]-1-propanesulfonic acid (CHAPS), 3-[(3-cholamidopropyl)dimethylammonio]-2-hydroxy-1-propanesulfonic acid (CHAPSO), palmitoyltoluidine, dodecyl-N-betaine and dodecyl-β-alanine; and nonionic surfactants such as octyl glucoside, peptyl thioglucoside, decanoyl-N-methylglucamide, polyoxyethylene dodecyl ether, polyoxyethylene heptamethylhexyl ether, polyoxyethylene isooctylphenyl ether (Triton X series), polyoxyethylene nonylphenyl ether, polyoxyethylene fatty acid ester, sucrose fatty acid ester and polyoxyethylene sorbitol ester (Tween series).

According to the invention, nonionic surfactants are used. One of them may be selected and used or two or more thereof may be selected and used jointly.

The effective amount for the stabilization of the surfactant to thrombin may be an amount for just improving the stability and, although there is no particular limitation, the final concentration of the surfactant when thrombin is in the dissolved liquid state is preferably 0.001~1 weight/volume % and, more preferably, 0.05~0.1 weight/volume %.

### (Protein)

With regard to the protein used as a stabilizing means for thrombin, there may be exemplified albumin, gelatin and globulin and those having no physiological or biochemical function to thrombin may be used in general. One of those proteins may be selected and used or two or more thereof may be selected and used jointly.

The effective amount for the stabilization of the protein to thrombin may be an amount for just improving the stability and, although there is no particular limitation, the final concentration of the protein when thrombin is in the dissolved liquid state is preferably 0.05~10 weight/volume % and, more preferably, 0.1~5 weight/volume %.

The above-mentioned calcium ion, water-soluble organic acid, surfactant and protein used as the means for the stabilization of thrombin are used by taking the influence of thrombin on enzymatic activity when thrombin is preserved in a state of solution, in a dry state or in a state where freeze-dried product is dissolved, etc. into consideration. Even when one of them is used solely, the stabilizing effect is available and, when two or more are selected and used, more stabilizing effect can be expected. It is preferred to use two or more members jointly. It is particularly preferred to use calcium ion and a water-soluble organic acid jointly whereby the stabilizing effect to thrombin resulted thereby is almost 100%. When two or more of the above-mentioned compounds having a stabilizing function to thrombin are used jointly, each of the compounds may be used within a usable range of each compound. Both in the case where each of the compounds is used solely and in the case where two or more are used jointly, the above-mentioned concentration(s) is/are contained in a liquid preparation when thrombin is a liquid preparation while, when thrombin is a dry preparation or a freeze-dried preparation, the compound(s) is contained after adjusting to such a manner that the final concentration(s) upon finally making into liquid is/are made the above-mentioned concentration(s).

### (Additives for Improving the Reproducibility of Measurement)

In addition to the above-mentioned means for the stabilization of thrombin, it is also possible that one or more which is/are selected from high-molecular polysaccharides and synthetic polymers is/are combined and its/their effective amount(s) is/are contained therein. In that case, the effective amount for reproducibility of the measurement may be an amount whereby the reproducibility can be improved and, although there is no particular limitation therefor, it is for example in such an amount that, when the substance to be tested is measured for 20 times using a reagent containing thrombin, a CV value (coefficient of variation) is able to be kept 10% or less, preferably 7% or less and, more preferably, 5% or less.

With regard to the high-molecular substances, there may be exemplified dextran 40, dextran 70, dextran 200,000, dextran 500,000 and Ficol and, with regard to the synthetic polymers, there may be exemplified polyvinyl alcohol 500, polyvinyl alcohol 1500, polyvinyl alcohol 2000, polyethylene glycol 1500, polyethylene glycol 2000, polyethylene glycol 4000, polyethylene glycol 6000, polyethylene glycol 8000, polyethylene glycol 20000 and polyvinylpyrrolidone. However, they are not limitative but there may used other substances which have the same action and are able to achieve an improvement in the measurement reproducibility for the test substance using a thrombin-containing reagent. With regard to those high-molecular substances, one of them may be selected and used or two or more may be selected and used jointly.

The effective amount for the reproducibility of the high-molecular polysaccharides or synthetic polymers may be an amount for just improving the reproducibility and, although there is no particular limitation, the final concentration of them when thrombin is in the dissolved liquid state is preferably 1~10 weight/volume % and, more preferably, 1~3 weight/volume %. The above-mentioned concentration(s) is/are contained in a liquid preparation of thrombin when thrombin is a liquid preparation while, when it is a dry preparation or a freeze-dried preparation, the compound(s) is contained after adjusting to such a manner that the final concentration (s) upon finallymaking into liquid is/are made the above-mentioned concentration(s).

### (Thrombin-Containing Composition)

The thrombin-containing composition contains one or more compound(s) having a thrombin-stabilizing function selected at least from calcium ion, surfactant and protein in thrombin in an amount effective for the stabilization of thrombin or it further contains a water-soluble organic acid having a thrombin-stabilizing effect in its effective amount. In addition to such compounds having a thrombin-stabilizing function, one or more compound(s) selected from high-molecular polysaccharides and synthetic polymers in effective amount (s) thereof may be contained therein so as to improve the reproducibility of the measurement of the substance to be tested using a thrombin-containing reagent. In the thrombin-containing composition, a buffer is usually used in liquid state. With regard to its type, a buffer having a buffering ability within a range of pH 4~9 is appropriately selected and used. There may be used, for example, one or more which is/are selected from HEPES, MOPS, BIS-TRIS, TRIS, MOPSO, ADA, MES and the like. An appropriate antiseptic agent may be added to the thrombin-containing composition as well. With regard to the preservative agent, one or more which is/are selected from ciprofloxacin, propionic acid, sodium benzoate, etc. may be used. If necessary, salt such as sodium chloride and common stabilizers such as amino acid and saccharide may also be contained therein.

The thrombin-containing composition prepared as such has a high stability as compared with the conventionally used thrombin reagents and compositions and its reproducibility in the measurement of the substance to be tested using a thrombin-containing reagent is good as well.

To be more specific, 5 mM to 100 mM of calcium ion such as calcium lactate are added to 5 mM to 25 mM of a buffer, then 10 mM to 500 mM of an organic acid such as acetic acid are added thereto and, after that, synthetic polymers such as polyvinyl alcohol 500 in an amount of 1~10 weight/volume % and/or surfactant such as Tween 80 in an amount of 0.001~1 % are further added thereto. Concentrations of those compounds to be added may be appropriately adjusted. After addition of the preservative agent, pH of the solution is adjusted to about 6~7 whereupon amother liquor is prepared. Into this mother liquor is dissolved thrombin in an aimed concentration or in an active amount to prepare a thrombin solution. The resulting solution may be filtered using a filter having an appropriate pore size. The thrombin solution prepared as such may be used as a liquid reagent as it is or, after subjecting to a freeze-drying treatment, it is made into a preparation as a freeze-dried reagent and is used. The above-mentioned specific examples just show the examples of the thrombin-containing composition and the present invention is not limited to such examples.

### Examples

The present invention will now be illustrated in more detail by way of the following examples although the present invention is not limited thereto.

### Example 1, not part of the claimed invention :

There were prepared four kinds of solutions, i.e. a 20 mM HEPES buffer (pH 6.0) (nothing-added solution); a solution prepared by adding 25 mM calcium lactate to the above buffer (Ca-added solution); a solution prepared by adding 100 mM acetic acid to the above buffer to adjust to pH 6.0 (acetic acid-added solution); and a solution prepared by adding 25 mM calcium lactate to the acetic acid-added solution (two substances-added solution) and to each of the solutions was added human thrombin so as to make its concentration 100 NIH units/ml whereupon a thrombin solution was prepared. To the thrombin solution prepared as such was added 0.1 weight/volume % of ciprofloxacin as an preservative agent, the mixture was subjected to a sterile filtration using a filter of 0.22 µm and each 3 ml of the mixture were placed in each glass bottle, tightly sealed and preserved at 25°C. The residual activity of thrombin was measured immediately after the preparation and after 1, 2, 3, 4, 5 and 6 month (s) in accordance with the Japanese Pharmacopoeia to check the stabilizing effect for thrombin. The result is shown in Table 1.

**Table 1**

| (Residual Activity of Thrombin; Preserved at 25°C) | | | | | | | |
|---|---|---|---|---|---|---|---|
| | Preserved Period | | | | | | |
| | Immediately after Preparation | After One Month | After Two Months | After Three Months | After Four Months | After Five Months | After Six Months |
| Nothing-Added | 100 | 89 | 84 | 57 | 35 | 17 | 8 |
| Ca-Added | 100 | 93 | 90 | 88 | 83 | 81 | 72 |
| Acetic Acid-Added | 100 | 97 | 93 | 89 | 85 | 82 | 74 |
| Two-Substances Added | 100 | 100 | 101 | 98 | 100 | 97 | 102 |

| | | | | | | | |
|---|---|---|---|---|---|---|---|
| (unit: %) | | | | | | | |

The result shows that, when at least calcium was added, thrombin was stabilized and, even after preserved at 25°C for six months, residual activity of 72% was maintained. Although the effect by addition of acetic acid is as same as that disclosed in Japanese Patent Laid-Open NO. 39782/1981, when calcium was further added in addition to acetic acid which is an organic acid, the stabilizing effect was further improved and, even after preserved at 25°C for six months, a decrease in the thrombin activity was not noted at all but a complete stabilization was found to be achieved.

### Example 2, not part of the claimed invention :

To 10 mM HEPES buffer (pH 7.0) were added 150 mM sodium chloride and 0.1 weight/volume % sodium azide and, after that, a solution where calcium lactate was added thereto so as to make its concentration 20 mM and a solution where calcium lactate was not added thereto were prepared. To each of the solutions was or was not added polyvinyl alcohol 205 (PVA 205), polyvinyl alcohol 500 (PVA 500) or polyvinyl alcohol 1500 (PVA 1500) to prepare solutions containing 0, 0.1, 0.5, 1, 2, 3 and 5 weight/volume % thereof.

Thrombin was dissolved in each of the solutions to make 100 NIH units/ml whereupon thrombin solutions were prepared. Each of the thrombin solutions was used for the measurement of fibrinogen concentration in a commercially available control plasma. The control plasma was diluted to an extent of 10-fold using a buffer for the measurement of fibrinogen to prepare a sample, 50 µl of the already-prepared thrombin solution were added to 100 µl of the said sample and the time until coagulation took place at 37°C was measured.

The above-mentioned series of operations were carried out using a fully automated coagulation analyzer (Coagrex 700; manufactured by Shimadzu). The same sample was measured for 20 times at the same time and, based on the resulting coagulation time, the coefficient of variation was determined and the reproducibility was compared. The result is shown in Table 2.

**Table 2**

| (Reproducibility of PVA-Added Thrombin Reagent) | | | | | | | | |
|---|---|---|---|---|---|---|---|---|
| 20 mM Calcium Lactate | Type of PVA Used | Polyvinyl Alcohol (PVA) Concentration | | | | | | |
| | | Not Added | 0.1% | 0.5% | 1% | 2% | 3% | 5% |
| Not Added | PVA 205 | 20.1 | 17.8 | 18.6 | 15.5 | 14.3 | 14.3 | 16.5 |
| | PAV 500 | 22.3 | 15.0 | 17.8 | 15.3 | 14.1 | 14.8 | 16.7 |
| | PAV 1500 | 20.6 | 19.3 | 17.5 | 14.7 | 15.2 | 14.2 | 17.8 |
| Added | PVA 205 | 13.6 | 9.0 | 8.6 | 8.6 | 4.3 | 4.9 | 6.5 |
| | PVA 500 | 16.3 | 5.0 | 7.8 | 5.0 | 4.1 | 4.8 | 6.7 |
| | PVA 1500 | 15.6 | 9.0 | 7.5 | 3.7 | 6.5 | 7.1 | 7.8 |

| | | | | | | | | |
|---|---|---|---|---|---|---|---|---|
| (unit: CV %) | | | | | | | | |

From Table 2, it is apparent that the coefficient of variation is smaller in thrombin solutions to which 20 mM of calcium lactate are added than the thrombin solutions to which no calcium lactate is added whereby the reproducibility is improved. It has been also found that, when polyvinyl alcohol is added, an improvement in reproducibility is achieved and the effect is maximum when it is 1~3 weight/volume %. From the results of Examples 1 and 2, calcium ion is found to have a stabilizing effect to thrombin and to be effective for an improvement in the reproducibility.

### Example 3, not part of the claimed invention :

The same operation as in Example 2 was carried out except that dextran 40 (Dex 40), dextran 70 (Dex 70) and dextran 200, 000 (Dex 200) were used instead of polyvinyl alcohol, that their concentrations were made 0.1, 0.5, 1, 3, 5, 6, 8 and 10 weight/volume % and that 20 mM calcium lactate were contained in all of them whereupon the reproducibility was compared. The result is shown in Table 3.

**Table 3**

| (Reproducibility of Dextran-Added but Non-Calcium-Added Thrombin Reagents) | | | | | | | | | |
|---|---|---|---|---|---|---|---|---|---|
| Type of Dex | Dextran (Dex) Concentration | | | | | | | | |
| | Not Added | 0.1% | 0.5% | 1% | 3% | 5% | 6% | 8% | 10% |
| Dex 40 | 13.6 | 21.0 | 12.8 | 9.9 | 4.8 | 3.4 | 3.4 | 3.7 | 4.3 |
| Dex 70 | 16.3 | 7.3 | 9.7 | 6.1 | 3.3 | 6.7 | 6.5 | 6.7 | 6.7 |
| Dex 200 | 15.6 | 20.1 | 14.2 | 8.8 | 4.6 | 5.8 | 5.5 | 6.3 | 7.0 |

| | | | | | | | | | |
|---|---|---|---|---|---|---|---|---|---|
| (unit: CV%) | | | | | | | | | |

It is noted from Table 3 that an improvement in the reproducibility is achieved when dextran which is a high-molecular polysaccharide is added and the effect is maximum at the concentrations of 1~10 weight/volume %.

### (Example 4)

As the samples, there were prepared nine kinds of solutions, i.e. 20 mM HEPES buffer pH 6.0) (non-added solution); a solution prepared by adding 25 mM calcium lactate to the said buffer to adjust to pH 6.0 (Ca-added solution); a solution prepared by adding 100 mM acetic acid to the said buffer to adjust to pH 6.0 (acetic acid-added solution); a solution prepared by adding 0.02 weight/volume % Tween 80 to the said buffer to adjust to pH 6.0 (Tween 80-added solution); a solution prepared by adding 1.0 weight/volume % bovine serum albumin to the said buffer to adjust to pH 6.0 (1% BSA); a solution prepared by adding 2.0 weight/volume % bovine serum albumin to the said buffer to adjust to pH 6.0 (2% BSA); a solution prepared by adding 0.1 weight/volume % aqueous gelatin to the said buffer to adjust to pH 6.0 (aqueous gelatin); a solution prepared by adding 25 mM calcium lactate to the above acetic acid-added solution to adjust to pH 6.0 (two-substances-added solution); and a solution prepared by adding 25 mM calcium lactate and 0.02 weight/volume % Tween 80 to the above acetic acid-solution to adjust to pH 6.0 (three-substances-added solution). To each of them was added human thrombin to make its concentration 100 NIH units/ml whereupon thrombin solutions were prepared.

To each of the thrombin solutions prepared as such was added 0.1 weight/volume % of ciprofloxacin as an antiseptic agent, the mixture was subjected to a sterile filtration using a filter of 0.22 µm and each 3 ml of the mixture were placed in each glass bottle, tightly sealed and preserved at 25°C. The residual activity of thrombin was measured immediately after the preparation and after 1, 2, 3, 4, 5 and 6 month (s) in accordance with the Japanese Pharmacopoeia to check the stabilizing effect for thrombin. The result is shown in Table 4.

**Table 4**

| (Residual Activity of Thrombin; Preserved at 25°C) | | | | | | | |
|---|---|---|---|---|---|---|---|
| | Preserved Period | | | | | | |
| | Immediately after Prepn | One Month | Two Months | Three Months | Four Months | Five Months | Six Months |
| Nothing Added | 100 | 89 | 79 | 57 | 35 | 17 | 8 |
| Ca-Added | 100 | 93 | 90 | 88 | 83 | 81 | 72 |
| Acetic Acid-Added | 100 | 97 | 93 | 89 | 85 | 82 | 74 |
| Tween 80-Added | 100 | 94 | 92 | 87 | 84 | 81 | 78 |
| 1% BSA-Added | 100 | 95 | 91 | 89 | 83 | 80 | 75 |
| 2% BSA-Added | 100 | 90 | 86 | 83 | 78 | 74 | 72 |
| 0.1% Water-Soluble Gelatin-Added | 100 | 91 | 85 | 83 | 78 | 75 | 73 |
| Two-Substances-Added | 100 | 100 | 101 | 98 | 101 | 97 | 102 |
| Three-Substances-Added | 100 | 102 | 98 | 101 | 100 | 101 | 100 |

| | | | | | | | |
|---|---|---|---|---|---|---|---|
| (unit: %) | | | | | | | |

The result is that, as compared with a solution containing no additive, stability of thrombin was improved by addition of Ca, aceticacid, Tween 80, 1% BSA, 2% BSA and water-soluble gelatin and that, in a solution where two substances (Ca and acetic acid) were added and a solution where three substances (Ca, acetic acid and Tween 80) were added, stability was further improved.

### Industrial Applicability

As fully illustrate hereinabove, thrombin was stabilized by addition of at least calcium and, as a result of supplementary addition of organic acid, a further stabilizing effect was achieved. It was furthermore found that surfactant and protein were useful for the stabilization of thrombin. In addition, calcium ion was found to be effective for an improvement in the reproducibility of measurement of thrombin activity together with a stabilizing effect to thrombin. Moreover, high-molecular polysaccharides and synthetic polymers were found to be useful for an improvement in the reproducibility of measurement of thrombin activity. Thus, a stabilizing means for thrombin and a thrombin-containing composition into which a stabilizing means is introduced in accordance with the present invention provide a stabilizing means which has not been available until now and provide an extremely useful reagent composition for clinical tests.

## Claims

1. Use of a nonionic surfactant and calcium ions in a thrombin reagent for clinical laboratory tests comprising thrombin, for improving the preservation stability of thrombin.

2. The use according to claim 1, wherein the nonionic surfactant is a polyoxyethylene sorbitol ester.

3. The use according to claim 1 or 2, wherein the final concentration of the nonionic surfactant is 0.001 to 1 w/v%.

4. The use according to any one of claims 1-3, **characterized in that** the reagent is liquid.

5. The use according to any one of claims 1-4, further containing an organic acid for improving the stability of thrombin.

6. The use according to any one of claims 1 to 5, **characterized in that** the reagent is suitable for measuring the concentration of fibrinogen.

7. A method for stabilizing thrombin in a thrombin reagent for clinical laboratory tests **characterized by** adding a nonionic surfactant and calcium ions to the reagent.

## Patentansprüche

1. Verwendung von einem nichtionischen oberflächenaktiven Mittel und Calcium-Ionen in einem Thrombin-Reagens für klinische Laborversuche, das Thrombin umfasst, um die Lagerungsstabilität von Thrombin zu verbessern.

2. Verwendung nach Anspruch 1, wobei das nichtionische oberflächenaktive Mittel ein Polyoxyethylensorbitol-Ester ist.

3. Verwendung nach Anspruch 1 oder 2, wobei die Endkonzentration des nichtionischen oberflächenaktiven Mittels 0.001 bis 1 w/v% ist.

4. Verwendung nach einem der Ansprüche 1 bis 3, **dadurch gekennzeichnet, dass** das Reagens flüssig ist.

5. Verwendung nach einem der Ansprüche 1 bis 4, wobei zusätzlich eine organische Säure zur Verbesserung der Stabilität von Thrombin enthalten ist.

6. Verwendung nach einem der Ansprüche 1 bis 5, **dadurch gekennzeichnet, dass** das Reagens zur Messung der Konzentration von Fibrinogen geeignet ist.

7. Verfahren zur Stabilisierung von Thrombin in einem Thrombin-Reagens für klinische Laborversuche, **gekennzeichnet durch** das Zugeben eines nichtionischen oberflächenaktiven Mittels und von Calcium-Ionen zu dem Reagens.

## Revendications

1. Utilisation d'un surfactant non ionique et d'ions de calcium dans un réactif à base de thrombine pour des tests cliniques en laboratoire comprenant de la thrombine, afin d'améliorer la stabilité de conservation de la thrombine.

2. Utilisation selon la revendication 1, dans laquelle le surfactant non ionique est un ester de polyoxyéthylène sorbitol.

3. Utilisation selon la revendication 1 ou 2, dans laquelle la concentration finale en surfactant non ionique va de 0,001 à 1 p/v%.

4. Utilisation selon l'une quelconque des revendications 1 à 3, **caractérisée en ce que** le réactif est liquide.

5. Utilisation selon l'une quelconque des revendications 1 à 4, contenant en outre un acide organique afin d'améliorer la stabilité de la thrombine.

6. Utilisation selon l'une quelconque des revendications 1 à 5, **caractérisée en ce que** le réactif est adapté pour mesurer la concentration de fibrinogène.

7. Procédé de stabilisation de la thrombine dans un réactif à base de thrombine pour des tests cliniques en laboratoire **caractérisé par** l'ajout d'un surfactant non ionique et d'ions de calcium au réactif.
